# EUROPEAN PATENT APPLICATION

(11) **EP 2 725 032 A1**
(43) Date of publication of application: **30.04.2014**
(21) Application number: 12306313.3
(22) Date of filing: 23.10.2012
(51) Int. Cl.: C07H 15/232

(54) **Highly pure tritiated paromomycin**

(71) Applicant: Ceva Sante Animale, 33500 Libourne Cedex (FR)
(72) Inventor: Magnier, Reynald, 33330 Saint Emilion (FR); Carrie, Aude, 33500 Les Billaux (FR)
(74) Representative: Tezier Herman, Béatrice

(57) **Abstract**

The present invention concerns a process for the purification of tritium marked paromomycin ([³H]paromomycin), highly pure tritiated paromomycin obtained by said process and a composition comprising said paromomycin. The present invention further concerns the use of said paromomycin for ecotoxicity tests.

## Description

The present invention concerns a process for the purification of tritium marked paromomycin ([³H]paromomycin), highly pure tritiated paromomycin obtained by said process and a composition comprising said paromomycin. The present invention preferably concerns the use of said tritium marked paromomycin for ecotoxicity tests in the veterinary field.

Paromomycin is a broad-spectrum aminoglycoside antibiotic produced by *Streptomyces Chrestomyceticus,* and structurally related to neomycin, streptomycin and kanamycin. Like other aminoglycosides, paromomycin appears to inhibit the protein synthesis in susceptible bacteria at the 30S subunit of the ribosome.

Paromomycin has a broad spectrum of activity, including activity against protozoa, bacteria and cestodes. The drug has an antibacterial spectrum similar to that of neomycin, but it is also active against some protozoa, including *Leishmania spp., Entamoeba histolytica* and *Cryptosporidium spp.,* and has anthelmintic properties against tapeworms. Paromomycin is believed to act against both the trophozoite and encysted forms of *Entamoeba histolytica.* The antibiotic is active against *Mycobacterium tuberculosis,* including multi-drug resistant strains and *Mycobacterium avium* complex. Paromomycin is bactericidal to many normal and pathogenic organisms in the gastrointestinal tract. Paromomycin is also considered as a luminal or contact amebicide since it acts principally in the intestinal lumen. Unlike tetracycline, paromomycin is a direct-acting amebicide and is effective either in the presence or absence of bacteria.

For example, paromomycin is used in the treatment of human intestinal protozoal infections, including acute and chronic amoebiasis, cryptosporidiosis in patients with AIDS, and giardiasis. Paromomycin has also been tried parenterally for visceral, and topically for cutaneous leishmaniasis. It has also been used in the treatment of tapeworm infection, but it is not the treatment of choice, and, similarly to neomycin, paromomycin is used in the suppression of intestinal flora both pre-operative treatment and management of hepatic encephalopathy.

Paromomycin is used as single oral dose to eliminate *Taenia saginata, Taenia solium* and *Diphyllobothrium latum,* and repeated doses are necessary to eliminate *Hymenolepis nana.*

In the veterinary field, paromomycin sulfate is intended for treatment of a number of bacterial infections (colibacillosis, salmonellosis) either by parenteral administration (calve, pigs, dry cows) or by oral administration in feed and drinking water (calves, piglets, broiler chickens).

Like other aminoglycosides, paromomycin and paromomycin sulfate are poorly absorbed after oral administration and most of the dose is eliminated unchanged in the faeces. After parenteral administration, accumulation occurs in the renal cortex and the cochlea and excretion in almost exclusively in the urine as unchanged drug.

As a result, mostly ingested antibiotic doses are excreted and subsequently disseminated into environmental compartments (such as water and soil).

Tritium and cabon-14 radiolabeled paromomycin and/or paromomycin sulfate are powerful tools for antibiotic tracer experiments in animals and environment.

Preparation of tritium-labelled paromomycin has initially been described in Ober R. et al. "Preparation of tritium-labelled paromomycin ('Humatin') by fermentation in a medium containing tritiated water", Nature, 1962 (193) :1289-90.

The synthesis method has been optimized by Capmau et al. ("A one pot tritiation of aminoglycoside antibiotics". J Antibiot, 1983, (36):735-737). In this method, tritium paromomycin was separated from other reaction components on a silica gel column. According to Griffiths et al, the achieved yields are about 25% and specific activities of about 1.14 x 10⁴ cpm/µg of paromomycin are obtained (Infection and Immunity, Aug. 1998, p. 3874-3883).

Tritium-labelled paromomycin can generally be obtained by fermentation in a solution containing tritiated reactants or with tritium gas. Purification of [³H]paromomycin is classically performed with HPLC using a typical C18 silica column. However, the HPLC retention time of paromomycin on C18 silica columns is very short. Therefore all materials, including paromomycin and impurities, elute at the solvent front. Consequently, paromomycin cannot be separated efficiently from impurities with such columns.

The Applicant discovered that the use of a porous graphite column for the purification of [³H]paromomycin surprisingly afforded very highly pure [³H]paromomycin in a high yield.

### Summary of the invention

The first object of the invention is a new process for purification of [³H]paromomycin, wherein the process comprises the use of a porous graphite column.

Another object of the invention is highly pure [3H]paromomycin obtained with the process of the invention.

Another object of the invention is a composition comprising highly pure [³H]paromomycin.

A fourth object of the invention is the use of the highly pure [³H]paromomycin or composition comprising the same for monitoring and/or identifying and/or quantifying paromomycin in tissues of animals or humans or in environment (preferably soil).

### Description of the figures

Figure 1: HPLC spectrum of [³H]paromomomycin obtained in example 2.
Figure 2: Table of the intensities of the spectrum of figure 1.
Figure 3: TOF (Time Of Flight) MS ES+ (electrospray) mass spectrum of [³H]paromomomycin obtained in example 2.
Figure 4: TOF MS ES+ mass spectrum of the inactive reference material.
Figure 5: Radioactive detection and UV detection of a) [³H] paromomycin according to the invention and b) commercial paromomycin analyzed with a porous graphite column.
Figure 6: a) TOF MS ES+ mass spectrum of [³H]paromomomycin according to the invention, b) TOF MS ES+ mass spectrum of commercial [³H]paromomomycin.

### Detailed description of the invention

The first object of the invention is a new process for purification of [³H]paromomycin, wherein the process comprises the use of a porous graphite column. Said purification aims at separating [³H]paromomycin from the other components obtained after its synthesis, such as free tritium or tritiated or non tritiated fragments or moieties of paromomycin. More particularly, the porous graphite column implemented in the process of the invention is a column used in liquid chromatography, more preferably HPLC (high performance liquid chromatography).

The [³H]paromomycin to be purified is prepared by any method known in the art. Tritiation of paromomycin is preferably performed with tritium gas, as described in (Wilzbach, K.E., J. Am. Chem. Soc., 79, 1013 (1957)).

The porous graphite column used in the process of the invention comprises porous graphite as stationary phase. Preferably, the porous graphite column consists of 100% porous graphite, without any linking phase. Among appropriate columns to be used in the process of the invention may be cited Hypercarb^{®} columns manufactured by Thermo Electron Corporation. The graphite particle size may be comprised between 2 and 8 µm. The graphite particle size is preferably about 3, 5 or 7 µm, in particular it is about 5 µm.

In an embodiment, the pore size of the porous graphite column is about 250 Å.

In the present invention, the term about refers to a range comprised between plus and minus 10% of the value.

The solvent system in the purification process of the invention, particularly liquid chromatography, and more particularly HPLC, comprises solvent A and solvent B. Solvent A is preferably trifluoroacetic acid in water, in particular 0.1% by volume trifluoroacetic acid in water, and solvent B is preferably trifluoroacetic acid in acetonitrile, in particular 0.1% by volume trifluoroacetic acid in acetonitrile.

The gradient used in the HPLC purification process of the invention is preferably 0% by volume B (i.e., 100% by volume A) for 10 minutes, and then 0 to 30% by volume B (i.e., 100 to 70% by volume A) over 30 minutes.

In an embodiment, the HPLC system used in the process of purification of the invention is the following:

| | |
|---|---|
| Column: | 100% porous graphite column (Hypercarb^{®}), 5 µm, 150 x 10 mm, |
| Solvent A : | 0.1% by volume trifluoroacetic acid in water, |
| Solvent B: | 0.1% by volume trifluoroacetic acid in acetonitrile, |
| Gradient: | 0% by volume B for 10 minutes, and then 0 to 30% by volume B over 30 minutes, |
| Flow rate: | 2 mL/minute, and |
| UV detection: | 230 nm. |

The process of purification of [³H]paromomycin according to the invention may comprise, before the use of the porous graphite column, a step of tritiation of paromomycin. It can also optionally comprise a step of chemical synthesis of paromomycin, before tritiation thereof.

The process of purification of [³H]paromomycin according to the invention affords highly pure [³H]paromomycin, with both a high chemical purity, and a high radiochemical purity.

Chemical purity can be assessed by any appropriate technique known in the art. Chemical purity can be assessed for instance by NMR (Nuclear magnetic Resonance), by mass spectrometry, preferably by TOF MS ES+, or by HPLC, preferably by HPLC, preferably using appropriate column and solvent system, for instance coupled to UV detection. Preferably, the chemical purity of [³H]paromomycin according to the invention is strictly superior to 95%, in particular superior or equal to 97%.

In the present invention, the percentage values are to be understood as mass percentages, unless otherwise specified.

Radiochemical purity (RCP) may be defined as the proportion of the total radioactivity in the sample which is present as the desired radiolabelled species. Radiochemical purity can be assessed by any appropriate technique known in the art. Radiochemical purity can be assessed for instance by HPLC, preferably using appropriate column and solvent system, for instance coupled to radioactive detection. Preferably, the radiochemical purity of [³H]paromomycin according to the invention is strictly superior to 95%, in particular superior or equal to 97%.

In an embodiment, the HPLC column used to assess the chemical and/or radiochemical purity of [³H]paromomycin is not a C18 silica column.

The chemical and/or radiochemical purity of [³H]paromomycin in the present invention is advantageously assessed by HPLC using a porous graphite column as described above, such as a Hypercarb^{®} column.

Another object of the invention is [³H]paromomycin, preferably highly pure [³H]paromomycin, obtained with the process of the invention. The terms "highly pure [³H]paromomycin" refer to pharmaceutical grade [³H]paromomycin. Pharmaceutical grade is a standard of purity meaning suitable for use as a medicine. As described above, the chemical purity of [³H]paromomycin according to the invention is preferably strictly superior to 95%, in particular superior or equal to 97%. As also described above, the radiochemical purity of [³H]paromomycin according to the invention is preferably strictly superior to 95%, in particular superior or equal to 97%.

In a particular embodiment, the present invention deals with a composition comprising, and preferably consisting of, [³H]paromomycin, with a radiochemical purity strictly superior to 95%, in particular superior or equal to 97%, and / or a chemical purity strictly superior to 95%, in particular superior or equal to 97%. Preferably, the composition presents a radiochemical purity strictly superior to 95%, in particular superior or equal to 97%, and a chemical purity strictly superior to 95%, in particular superior or equal to 97%.

The chemical impurities may be tritiated or non tritiated fragments or moieties of paromomycin, or secondary products due to the process used for the synthesis of paromomycin. The radiochemical impurities are tritiated impurities, such as any [³H] chemical fragments or moieties of paromomycin.

The composition comprising, and preferably consisting of, [³H]paromomycin of the invention may be obtained by the process of purification as described above.

In an embodiment, the mass spectrum, preferably the TOF MS ES+ mass spectrum, of [³H]paromomycin or of the composition of the invention comprises, as only significant m/z peaks, for instance as only peaks of intensity higher than 5%, preferably higher than 10% , those corresponding to 308.65, 309.15, 309.65 and 616.31. The combination of these m/z peaks is the signature of tritiated paromomycin.

The present invention also concerns a process for the analysis of a [³H]paromomycin sample, comprising the use a porous graphite HPLC column as described above, such as a Hypercarb^{®} column.

Another object of the invention is the use of [³H]paromomycin or the composition comprising the same, as defined above, as a tool for ecotoxicity tests or as a research tool. More specifically, it is useful as a tool for studying the behavior of [³H]paromomycin in the environment, in particular in soil, in water and/or in the air. These tests allow assessing the environmental risk of paromomycin. Indeed, the aims of ecotoxicity tests performed for veterinary or human, preferably veterinary, medicinal products are to assess their potential harmful effects on the environment and/or to identify any precautionary measures which may be necessary to reduce said harmful effects. In the context of veterinary medicinal products, such precautionary measures include, but are not limited to, controlling the application rate, the amount of contaminated manure being spread on agricultural lands or the access of treated pasture animals to surface waters. Such precautionary measures are aimed at mitigating the environmental impact of the use of veterinary medicinal product. Ecotoxicity studies are necessary for instance when applying for marketing authorization for a veterinary medicinal product, such as paromomycin.

Tritium labeling of paromomycin according to the present invention allows an easy detection of paromomycin in the soil, water, air, and in various organisms, in particular aquatic organisms or organisms to which it is not intended (i.e., other non-target organisms). More particularly, tritium labeling of paromomycin according to the present invention allows monitoring the levels of excreted paromomycin into the environment by treated animals, and persistence thereof in such excretia. Tritium labeling of paromomycin according to the present invention also allows monitoring the levels of unused or wasted paromomycin spread into the environmental systems, and persistence thereof in such environmental systems.

Ecotoxicity tests that can be performed according to the invention comprise studying the likely extent to which paromomycin (more specifically non-excreted or excreted paromomycin) will enter into environmental systems, in particular into the soil, water and/or air, its effects on aquatic organisms, and/or its effects on other organisms, in particular organisms to which paromomycin is not intended. Among the organisms to which the drug is not intended may be cited humans and animals, preferably animals, in particular non-human mammals. Among animals may be cited ruminants and fowl, preferably ruminants. For instance, the non-human mammals are cattle, preferably dairy cattle. Where the conclusions of these studies indicate potential exposure of the environment to the product, evaluation of the potential ecotoxicity of the product is performed. For this purpose, it shall be considered the extent and duration of exposure of the environment to the product, as well as the information about the physical/chemical, pharmacological and/or toxicological properties of the compound obtained during the conduct of the other tests and trials required by health authorities.

An *in vitro* or *ex vivo* method for monitoring and/or identifying and/or quantifying paromomycin, can comprise the following steps : obtaining a sample susceptible to be radiolabelled by [³H]paromomycin of the invention ; and determining the level of radioactivity of said sample, in particular by liquid scintillation counting (LSC) ; and optionally identifying and/or quantifying paromomycin by using an appropriate method, such as for example a liquid chromatography tandem mass spectrometry (LC-MS/MS) procedure, optionally with radiometric detection.

More specifically, according to the method of the invention, [³H]paromomycin of the invention was previously spread into the environment of interest or administered to an animal or a patient. The method of the invention allows therefore monitoring the levels of unused, wasted or excreted paromomycin into the environment, and persistence thereof in such environment. The sample susceptible to be radiolabelled by [³H]paromomycin of the invention can be from an animal or a patient to which [³H]paromomycin of the invention was previously administered; in this context, the sample can be various tissues, urine sample, plasma sample or excretory (faeces) sample. The sample can also be from the environmental system or a sample from an aquatic organism or a sample from another non-target organism.

The following examples are intended to be illustrative, and not limitative, of the present invention.

### Examples

### Example 1 : Preparation of [³H]paromomycin

60 mg paromomycin sulphate was dissolved in 1 mL 0.1 M sodium phosphate buffer at pH 7. 30 mg of PdO on baryum sulphate were added and the mixture was stirred for 3 hours under 10 Ci tritium gas. After 3 hrs, the reaction mixture was filtered via a 0.45 µm GMF filter. The filtrate was dried by rotary evaporation and re-constituted in 0.75 mL ethanol: water (1 :1). Labile tritium was removed by rotary evaporation. The process was repeated 3 times to ensure complete removal of labile tritium. The product was dissolved in 8 mL water. The radiochemical yield of the crude product was 36 mCi.

### Example 2 : Purification of [³H]paromomycin

The crude product obtained in example 1 was purified in 4 lots of 2 mL, using the following HPLC system:

| | |
|---|---|
| Column: | Hypercarb^{®} 5 µ, 150 x 10 mm |
| Solvent A: | 0.1% trifluoroacetic acid in water |
| Solvent B: | 0.1% trifluoroacetic acid in acetonitrile |
| Gradient: | 0% B for 10 minutes, and from 0 to 30% B over 30 minutes |
| Flow rate: | 2 mL/minute |
| UV detection: | 230 nm |

The [³H]paromomycin fractions from the 4 HPLC runs were pooled, dried by rotary evaporation and re-constituted in 5 mL water.

The radiochemical yield of the pure product was 11 mCi.

### Example 3 : Analysis of [³H]paromomycin

The radiochemical purity of [³H]paromomycin was determined by HPLC in the following system:

| | |
|---|---|
| Column: | Hypercarb^{®} 100 x 4.6 mm, 5 µm |
| Column temperature: | 35°C |
| Eluent A: | 0.1 % trifluoroacetic acid (aqueous) |
| Eluent B: | 0.1% trifluoroacetic acid in acetonitrile |
| Gradient/flow rate: | 1% B to 50% B over 30 minutes, hold for 2 minutes, at 0.5 mL/minute. |

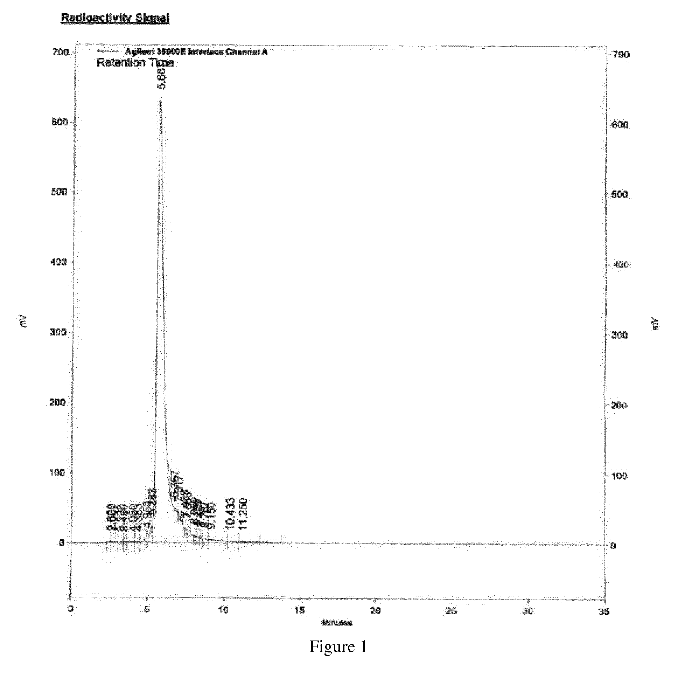

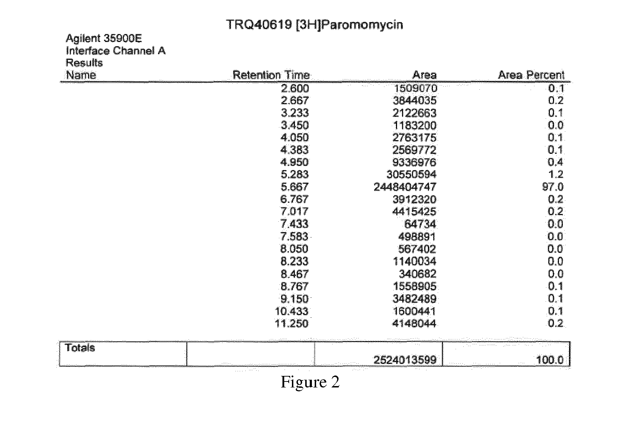

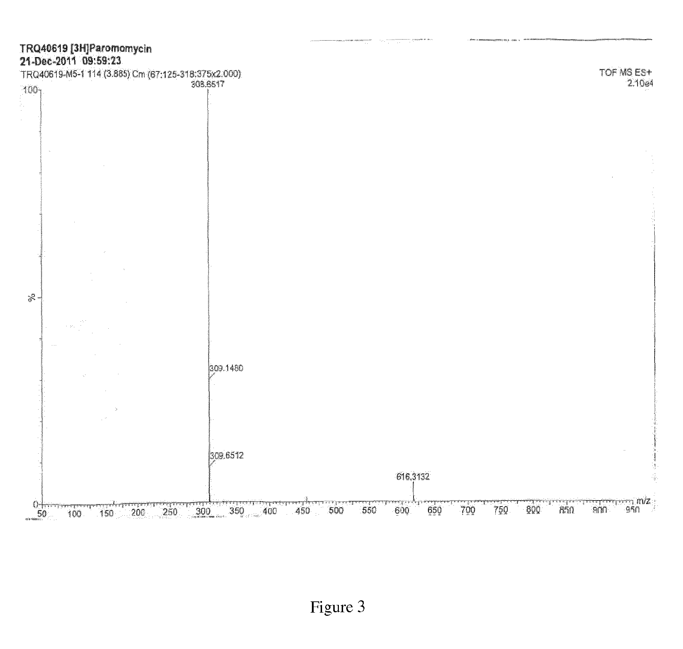

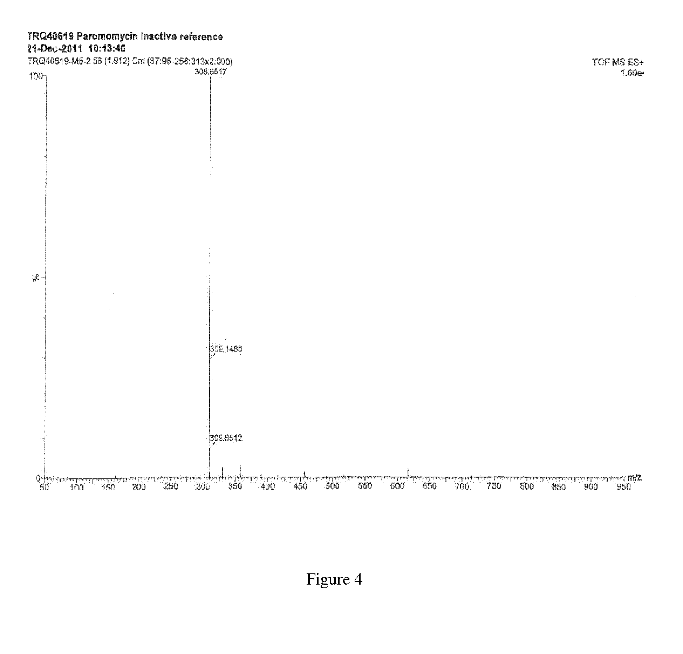

The radiochemical purity assessed by HPLC was 97.0% (see figures 1 and 2). The mass-spectrometry of [³H]paromomycin obtained in example 2 gave a spectrum that was consistent with the inactive material (see figures 3 and 4), and a specific activity of 0,6 Ci/mmol.

### Example 4: Dilution and packaging of [³H]paromomycin

[³H]paromomycin obtained according to example 2 was diluted to 1 mCi/mL with water, and packaged as 2 x 1 mCi and 1x8 mCi packs.

### Example 5: Comparative example

A sample of [³H] paromomycin purified according to the invention and a commercial sample of [³H] paromomycin sold by Moravek as having a purity higher or equal to 95% were analyzed both on a C18 silica column and on a porous graphite column (Hypercarb^{®}).
- The analysis on the C18 silica column afforded a purity higher than 95% for both compounds;
- The analysis on the porous graphite column afforded a radiochemical purity of more than 96% for the sample according to the invention, and a radiochemical purity around 2% for the commercial sample.

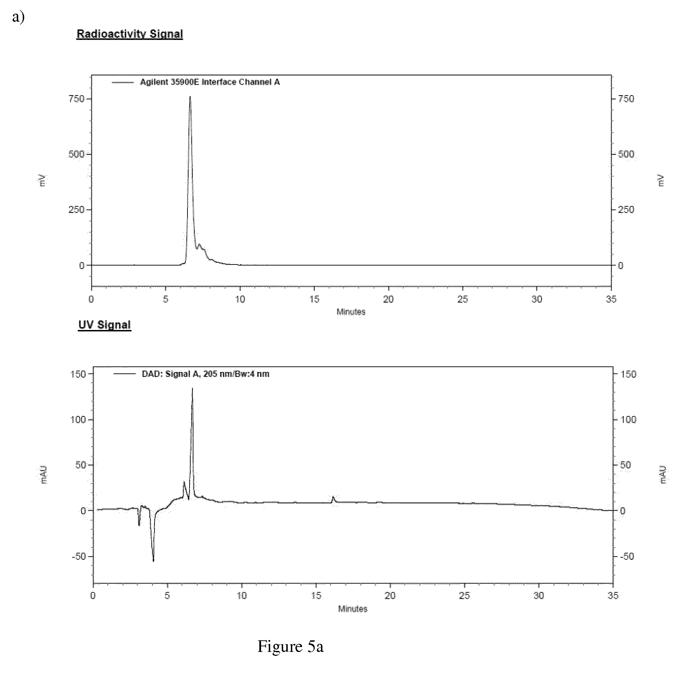

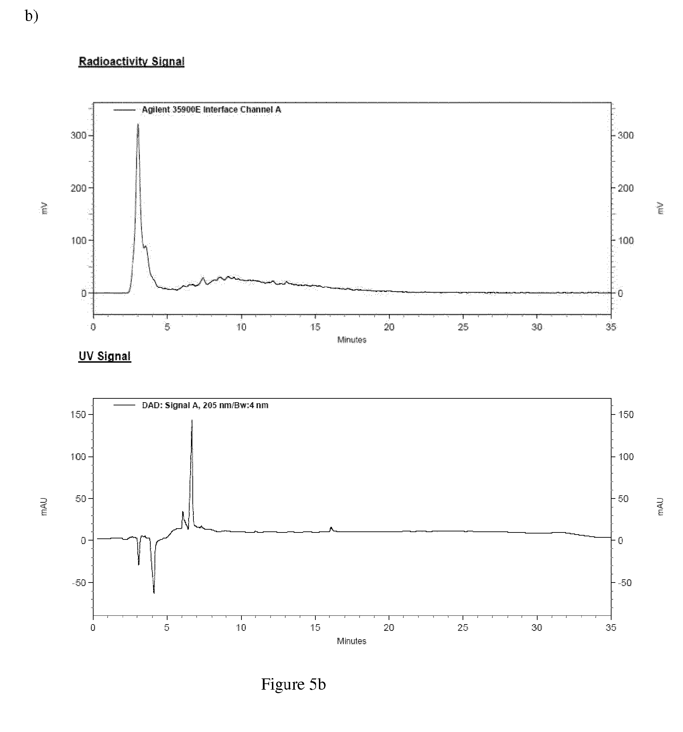

Figure 5 presents the radioactive detection and UV detection (205 nm) after the analysis of both samples with a porous graphite column (Hypercarb^{®}). The conditions for the analysis are the following:
- Column: Hypercarb^{®} 100 x 4.6 - 5 µm
- Column temperature: 35°C
- Eluant A: 0.1% trifluoroacetic acid (aqueous)
- Eluant B: 0.1% trifluoroacetic acid in acetonitrile
- Gradient/flow rate: 1%B-50%B over 30 minutes, hold for 2 minutes at 0.5 mL/min.

Both UV and radioactive detection show the same retention time for the main peak of the sample according to the invention, which shows that [³H]paromomycin is both chemically pure and radiochemically pure.

Conversely, the main peak in UV detection for the commercial sample probably corresponds to paromomycin, but the intensity of the signal for the same retention time in the radioactive detection is clearly low. Consequently, the majority of the tritiated species in the sample is not paromomycin.

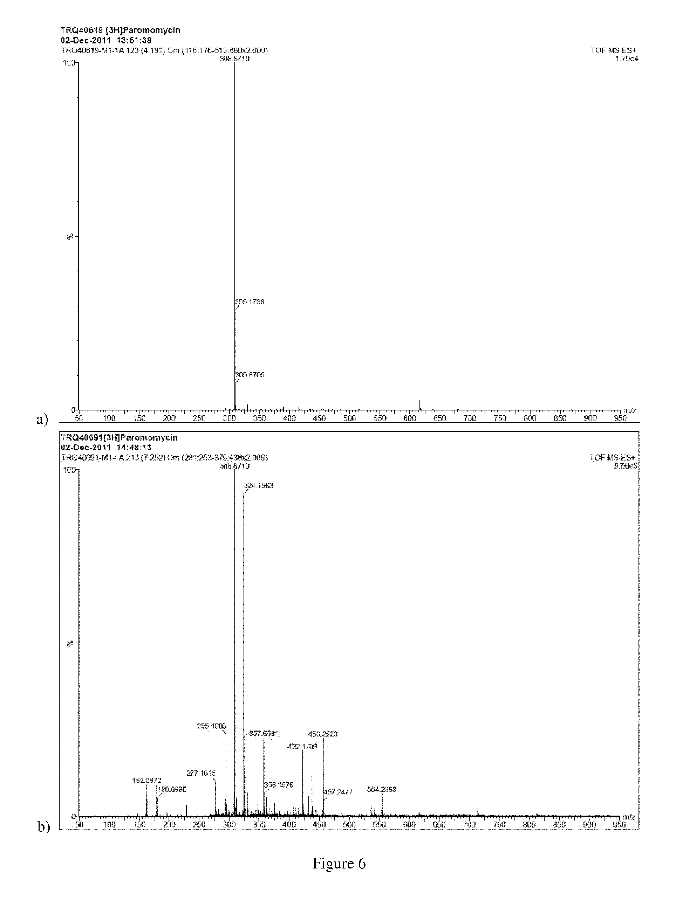

Figure 6 presents the mass spectra of both samples, proving that the commercial sample is a complex mixture, when the sample according to the invention is chemically pure. Consequently, the purification process of the invention is clearly more powerful than the purification system classically used for [³H] paromomycin.

## Claims

1. Process for purification of [³H]paromomycin, wherein the process comprises the use of a porous graphite column.

2. Process according to claim 1, wherein the porous graphite column is the column used in liquid chromatography.

3. Process according to claim 2, wherein the column is used in HPLC.

4. Process according to any of claims 1 to 3, wherein the porous graphite column consists of 100% porous graphite.

5. Process according to anyone of the preceding claims, wherein the column graphite particle size is 5 µm.

6. Process according to any of claims 2 to 5, wherein the HPLC system is the following:
Column: 100% porous graphite column, 5 µm, 150 x 10 mm,
Solvent A: 0.1% trifluoroacetic acid in water,
Solvent B: 0.1% trifluoroacetic acid in acetonitrile,
Gradient: 0% B for 10 minutes, and then 0 to 30% B over 30 minutes,
Flow rate: 2 mL/minute, and
UV detection: 230 nm.

7. Composition comprising [³H]paromomycin obtained by the process according to any of claims 1 to 6.

8. Composition comprising [³H]paromomycin with a chemical purity strictly superior to 95%, in particular superior or equal to 97%, and a radiochemical purity strictly superior to 95%, in particular superior or equal to 97%.

9. Use of the composition according to claim 7 or 8 as a tool for at least one ecotoxicity test.

10. Use according to claim 9, for monitoring and/or identifying and/or quantifying paromomycin in the environment.

11. Use according to claim 10, for monitoring and/or identifying and/or quantifying paromomycin in the soil or in various organisms.

12. Use according to claim 9, for monitoring and/or identifying and/or quantifying paromomycin in non human mammals.

13. Use according to claim 12, wherein non human mammals are cattle, preferably dairy cattle.

14. Use according to claim 8, for monitoring and/or identifying and/or quantifying paromomycin in aquatic organisms, and/or in other organisms to which it is not intended.
